(19) Europäisches Patentamt
European Patent Office
Office européen des brevets

(11) Veröffentlichungsnummer: **0 173 191**
**A2**

(12) # EUROPÄISCHE PATENTANMELDUNG

(21) Anmeldenummer: **85110314.3**

(22) Anmeldetag: **17.08.85**

(51) Int. Cl.[4]: **C 07 D 239/54,** C 07 D 239/30

(30) Priorität: **29.08.84 DE 3431698**

(43) Veröffentlichungstag der Anmeldung: **05.03.86 Patentblatt 86/10**

(84) Benannte Vertragsstaaten: **CH DE FR GB LI**

(71) Anmelder: **BAYER AG, Konzernverwaltung RP Patentabteilung, D-5090 Leverkusen 1 Bayerwerk (DE)**

(72) Erfinder: **Beitzke, Bernhard, Dr., Bergmannsweg 5, D-5060 Bergisch-Gladbach (DE)**
Erfinder: **Blank, Heinz Ulrich, Dr., Am Geusfelde 35, D-5068 Odenthal (DE)**

(54) **5,5-Dichlor-4,5-dihydro-6-hydroxy-2-trichlormethyl-pyrimidin-4-on, ein Verfahren zu seiner Herstellung und seine Verwendung.**

(57) Das neue 5,5-Dichlor-4,5-dihydro-6-hydroxy-2-trichlormethyl-pyrimidin-4-on kann durch Umsetzung von 4,6-Dihydroxy-2-methylpyrimidin oder chlorierter Folgestufen mit einem Chlorierungsmittel hergestellt werden. 5,5-Dichlor-4,5-dihydro-6-hydroxy-2-trichlormethyl-pyrimidin-4-on kann als Zwischenprodukt für die Herstellung von 2-Trichlormethyl-4,5,6-trichlorpyrimidin verwendet werden.

EP 0 173 191 A2

BAYER AKTIENGESELLSCHAFT 5090 Leverkusen, Bayerwerk
Konzernverwaltung RP Mn/by-c
Patentabteilung

# 5,5-Dichlor-4,5-dihydro-6-hydroxy-2-trichlormethyl-pyrimidin-4-on, ein Verfahren zu seiner Herstellung und seine Verwendung

Die Erfindung betrifft 5,5-Dichlor-4,5-dihydro-6-hydroxy-2-trichlormethyl-pyrimidin-4-on, ein Verfahren zu seiner Herstellung und seine Verwendung zur Herstellung von 2-Trichlormethyl-4,5,6-trichlorpyrimidin.

Das 2-Trichlormethyl-4,5,6-trichlorpyrimidin der Formel

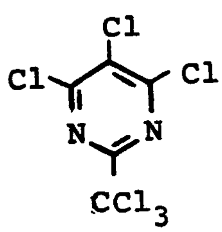

kann aus 2-Cyanoethylaminen durch Chlorierung hergestellt werden (C.A. 74, 100079d). Bei diesem Verfahren erhält man 2-Trichlormethyl-4,5,6-trichlorpyrimidin nur in Ausbeuten von maximal 60 %. Außerdem entstehen hierbei unerwünschte Nebenprodukte wie z.B. Hexachlorethan.

Es wurde das neue 5,5-Dichlor-4,5-dihydro-6-hydroxy-2-trichlormethyl-pyrimidin-4-on gefunden.

5,5-Dichlor-4,5-dihydro-6-hydroxy-2-trichlormethyl-pyrimidin-4-on kann durch die Formel

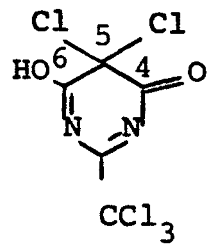

beschrieben werden.

Selbstverständlich kann das erfindungsgemäße 5,5-Dichlor-4,5-dihydro-6-hydroxy-2-trichlormethyl-pyrimidin-4-on auch in einer seiner tautomeren Formen vorliegen.

Die neue Verbindung ist ein Zwischenprodukt für die Herstellung von 2-Trichlormethyl-4,5,6-trichlorpyrimidin. Über das Zwischenprodukt kann das 2-Trichlormethyl-4,5,6-trichlorpyrimidin in hohen Ausbeuten und ohne unerwünschte Nebenprodukte hergestellt werden.

Es wurde auch ein Verfahren zur Herstellung von 5,5-Dichlor-4,5-dihydro-6-hydroxy-2-trichlormethyl-pyrimidin-4-on gefunden, das dadurch gekennzeichnet ist, daß man 4,6-Dihydroxy-2-methylpyrimidin oder chlorierte Folgestufen mit einem Chlorierungsmittel umsetzt.

4,6-Dihydroxy-2-methylpyrimidin der Formel

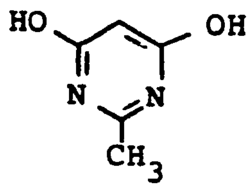

ist an sich bekannt (J. Org. Chem. 17, 1320 bis 1321 (1952)). Es kann beispielsweise durch Umsetzung von Acetamidin mit Malonsäurediethylester in Gegenwart von Natrium-ethylat hergestellt werden.

Chlorierte Folgestufen des 4,6-Dihydroxy-2-methyl-pyrimidins sind solche mit 1 bis 4 Chloratomen. Beispielsweise sei hier das bekannte 5-Chlor-4,6-dihydroxy-2-methyl-pyrimidin der Formel

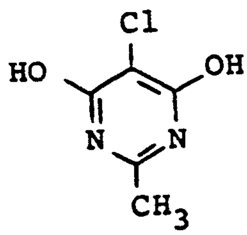

genannt (J. Org. Chem. 26, 1874 bis 1877 (1961)). Bevorzugte Ausgangsstoffe für die Herstellung des erfindungsgemäßen 5,5-Dichlor-4,5-dihydro-6-hydroxy-2-trichlormethyl-pyrimidin-4-ons sind das 4,6-Dihydroxy-2-methylpyrimidin und das 5-Chlor-4,6-dihydroxy-2-methyl-pyrimidin. Insbesondere bevorzugt wird 4,6-Dihydroxy-2-methylpyrimidin.

Chlorierungsmittel für das erfindungsgemäße Verfahren sind beispielsweise elementares Chlor, Sulfurylchlorid,

Chloramine oder Chloramide wie N-Chlorsuccinimid, ein Gemisch aus Chlorwasserstoff und Wasserstoffperoxid, Iodchlorid, Bromchlorid und Säurechloride wie Phosphorpentachlorid. Bevorzugte Chlorierungsmittel sind elementares Chlor und Sulfurylchlorid. Insbesondere bevorzugt wird Chlor.

Das Chlorierungsmittel wird vorzugsweise in zumindest stöchiometrischer Menge bezogen auf das eingesetzte 4,6-Dihydroxy-2-methyl-pyrimidin bzw. die chlorierten Folgestufen eingesetzt. Im allgemeinen setzt man 5 bis 7 Mol, bevorzugt 5,1 bis 6 Mol des Chlorierungsmittels bezogen auf das 4,6-Dihydroxy-2-methylpyrimidin ein.

Die erfindungsgemäße Chlorierung kann in Gegenwart von Katalysatoren durchgeführt werden. Als solche seien beispielsweise genannt:

- Amine (z.B. primäre, sekundäre, tertiäre aliphatische Amine, wie Triethylamin, N,N-Diisopropyl-N-ethylamin, 1,8-Bis-(dimethyl-amino)-naphthalin, 1,4-Diazabicyclo[2,2,2]octan (DABCO), N-Methylpiperidin, N-Methylpyrrolidin, heterocyclische Amine wie z.B. Pyridin, Lutidin, Collidin, Chinolin, Acridin, 4-Dimethylaminopyridin, Imidazol, N-Methyl-imidazol, Amidine wie Formamidin, 1,5-Diazabicyclo[4,3,0]non-5-en (DBN) oder 1,8-Diazabicyclo[5,4,0]undec-7-en (DBU)),

- Phosphorverbindungen (wie Triphenylphosphin, Triphenylphosphinoxid, Phosphortrichlorid und Phosphorpentachlorid),

- Carbonsäureamide (wie Dimethylformamid, Dimethylacetamid und N-Methylpyrrolidon, Phthalimid oder Succinimid),

- Nitrile (wie Acetonitril und Benzonitril),

- Friedel-Crafts-Katalysatoren (wie Aluminiumchlorid, Eisentrichlorid, Galliumtrichlorid, Zinkchlorid, Kupferdichlorid, Zinntetrachlorid, Titantetrachlorid),

- Iod oder Schwefel, und

- Radikalbildner (Peroxide wie z.B. Dibenzoylperoxid und Azoverbindungen wie z.B. Azo-bis-(isobutyronitril)).

Bevorzugte Katalysatoren sind: Triethylamin, Phosphortrichlorid, Eisen (III)chlorid, Dimethylformamid, Iod, Benzoylperoxid.

Der Katalysator wird im allgemeinen in einer Menge von 0,01 bis 5 Mol-%, bevorzugt 0,1 bis 3 Mol-%, bezogen auf die Ausgangsverbindung, eingesetzt.

Das erfindungsgemäße Verfahren wird bevorzugt in Gegenwart eines Lösungs- oder Suspensionsmittels durchgeführt. Lösungs- oder Suspensionsmittel für das erfindungsgemäße Verfahren sind unter den Reaktionsbedingungen flüssige Verbindungen, die gegen Chlor inert sind und unter den Reaktionsbedingungen nicht mit den Ausgangsverbindungen reagieren. Beispielsweise seien chlorierte Kohlenwasserstoffe wie Chlorbenzol, die isomeren Dichlorbenzole, die

isomeren Trichlorbenzole, Tetrachlorkohlenstoff, Chloroform und Hexachlorethan, Fluorchlorkohlenwasserstoffe wie 1,1,2-Trichlor-1,2,2-trifluorethan, Benzol, Phosphortrichlorid oder Phosphoroxychlorid genannt. Bevorzugte Lösungs- oder Suspensionsmittel sind die chlorierten Benzole und Phosphoroxychlorid.

Die Chlorierung kann sowohl zu einer Suspension als auch zu einer Lösung des 5,5-Dichlor-4,5-dihydro-6-hydroxy-2-trichlormethylpyrimidin-4-ons führen. Die Konzentration des Edukts kann so gewählt werden, daß am Ende der Chlorierung eine Lösung oder auch eine Suspension vorliegt. Die Konzentrationen des Eduktes liegen bevorzugt zwischen 0,5 und 8 Mol/l Lösungs- bzw. Suspensionsmittel.

Die Chlorierung kann auch unter Bestrahlung mit Licht durchgeführt werden. Im allgemeinen verwendet man Licht der Wellenlänge 240 bis 750 nm, bevorzugt 320 bis 500 nm.

Die erfindungsgemäße Chlorierung wird im allgemeinen im Temperaturbereich von 20 bis 180°C, bevorzugt von 30 bis 160°C, besonders bevorzugt von 30 bis 120°C, durchgeführt.

In Phosphoroxychlorid als Lösungs- bzw. Suspensionsmittel ist es zweckmäßig, zwischen 30 und 70°C zu chlorieren, damit noch keine Reaktion an den Oxogruppen in 4- und/oder 6-Position stattfindet.

In den chlorierten Benzolen wird bevorzugt zwischen 60 und 160°C chloriert.

Das erfindungsgemäße Verfahren wird im allgemeinen bei Normaldruck durchgeführt. Es ist aber auch möglich bei Unter- oder Überdruck (beispielsweise im Druckbereich von 0,1 bis 10 bar) zu chlorieren.

Das erfindungsgemäße 5,5-Dichlor-4,5-dihydro-6-hydroxy-2-trichlormethyl-pyrimidin-4-on kann zur Herstellung von 2-Trichlormethyl-4,5,6-trichlorpyrimidin verwendet werden. Die Herstellung des 2-Trichlormethyl-4,5,6-trichlorpyrimidins ist dadurch gekennzeichnet, daß man das 5,5-Dichlor-4,5-dihydro-6-hydroxy-2-trichlormethyl-pyrimidin-4-on mit einem Säurechlorid und gegebenenfalls in Gegenwart eines Halogens der Formel

XCl

worin

X Chlor, Brom oder Iod bedeutet,

und gegebenenfalls in Gegenwart eines Katalysators umsetzt.

Säurechloride sind im allgemeinen Phosphoroxychlorid, Thionylchlorid, Phosgen, Oxalylchlorid, Phosphortrichlorid, Phosphorpentachlorid, Dichlorphosphorane, Schwefeldichlorid und Dischwefeldichlorid.

Bevorzugte Säurechloride sind Phosgen, Phosphoroxychlorid und/oder Phosphorpentachlorid bzw. Phosphortrichlorid und Chlor.

Besonders bevorzugt verwendet man Phosphoroxychlorid und/ oder Phosphorpentachlorid bzw. Phosphortrichlorid und Chlor.

Das Säurechlorid wird im allgemeinen in einer Menge von 2 bis 3 Mol, bevorzugt von 2 bis 2,5 Mol, bezogen auf ein Mol 5,5-Dichlor-4,5-dihydro-6-hydroxy-2-trichlormethyl-pyrimidin-4-on, eingesetzt.

Die Umsetzung kann gegebenenfalls in Gegenwart eines Halogens durchgeführt werden. Als Halogene seien Chlor, Bromchlorid oder Iodchlorid, bevorzugt Chlor genannt.

Die Halogene werden im allgemeinen in einer Menge von 0 bis 3 Mol, bevorzugt von 0 bis 2 Mol, bezogen auf ein Mol 5,5-Dichlor-4,5-dihydro-6-hydroxy-2-trichlormethyl-pyrimidin-4-on, eingesetzt.

Die Herstellung des 2-Trichlormethyl-4,5,6-trichlorpyrimidins wird im allgemeinen im Temperaturbereich von 40 bis 200°C, bevorzugt von 50 bis 180°C, besonders bevorzugt von 60 bis 140°C, durchgeführt.

Die Herstellung des 2-Trichlormethyl-4,5,6-trichlorpyrimidins wird im allgemeinen bei Normaldruck durchgeführt. Es ist aber auch möglich, die Umsetzung bei Unter- oder Überdruck (beispielsweise im Druckbereich von 0,1 bis 10 bar) durchzuführen.

Das erfindungsgemäße Verfahren kann gegebenenfalls in Gegenwart eines Katalysators durchgeführt werden. Es können in der Regel die gleichen Katalysatoren verwendet werden, die bei der Chlorierung eingesetzt werden können.

Die Herstellung des 2-Trichlormethyl-4,5,6-trichlorpyrimidins wird bevorzugt in einem Lösungs- oder Suspensionsmittel durchgeführt. Als Lösungs- oder Suspensionsmittel können in der Regel die gleichen verwendet werden, die bei der Chlorierung eingesetzt werden können.

Die Herstellung des 5,5-Dichlor-4,5-dihydro-6-hydroxy-2-trichlormethyl-pyrimidin-4-ons und des 2-Trichlormethyl-4,5,6-trichlorpyrimidins kann beispielsweise wie folgt durchgeführt werden:

Man legt das 4,6-Dihydroxy-2-methylpyrimidin, gegebenenfalls in einem Lösungs- bzw. Suspensionsmittel und gegebenenfalls in Gegenwart des Katalysators, vor und dosiert das Chlorierungsmittel zu. Selbstverständlich kann man

auch das 4,6-Dihydroxy-2-methylpyrimidin und das Chlorierungsmittel simultan zu einem Lösungs- bzw. Suspensionsmittel eindosieren. Das 5,5-Dichlor-4,5-dihydro-6-hydroxy-2-trichlormethyl-pyrimidin-4-on wird nach der Umsetzung durch Absaugen isoliert und kann dann weiter zu dem 2-Trichlormethyl-4,5,6-trichlorpyrimidin umgesetzt werden.

Für diese Umsetzung legt man das 5,5-Dichlor-4,5-dihydro-6-hydroxy-2-trichlormethyl-pyrimidin-4-on in einem Lösungs- bzw. Suspensionsmittel vor und versetzt mit dem Säurechlorid. Man läßt solange reagieren, bis die Gasentwicklung beendet ist.

In einer bevorzugten Ausführungsform des erfindungsgemäßen Verfahrens erfolgt die Herstellung des 2-Trichlormethyl-4,5,6-trichlorpyrimidins aus dem 4,6-Dihydroxy-2-methylpyrimidin oder einer chlorierten Folgestufe ohne Isolierung des 5,5-Dichlor-4,5-dihydro-6-hydroxy-2-trichlormethyl-pyrimidin-4-ons in einer Eintopfreaktion. In dieser bevorzugten Ausführungsform wird zu dem Reaktionsgemisch der ersten Stufe das Säurechlorid hinzugegeben.

Es ist aber auch möglich, 4,6-Dihydroxy-2-methylpyrimidin oder eine seiner chlorierten Folgestufen in der ersten Stufe mit einer unterstöchiometrischen Menge (beispielsweise wenigstens 1 Mol bezogen auf die Ausgangsverbindung) Chlor oder chlorabspaltender Verbindung umzusetzen und das erhaltene Reaktionsgemisch in der zweiten Stufe

mit einem Säurechlorid und der erforderlichen Restmenge an Chlor oder chlorabspaltender Verbindung umzusetzen.

In einer besonders bevorzugten Ausführungsform wird das Reaktionsgemisch aus der ersten Stufe, das den Katalysator schon enthalten kann, mit einer Teilmenge Phosphortrichlorid versetzt, worauf die Restmenge Phosphortrichlorid und das gesamte noch notwendige Chlor simultan eingeleitet werden. Nach beendeter Dosierung wird noch solange erhitzt, bis die Gasentwicklung beendet ist.

Die Isolierung des 2-Trichlormethyl-4,5,6-trichlorpyrimidins erfolgt im allgemeinen durch Destillation.

2-Trichlormethyl-4,5,6-trichlorpyrimidin ist ein Zwischenprodukt für Farbstoffe (DE-OS 23 25 602 und DE-OS 23 26 112). Es kann außerdem als Zwischenprodukt für Herbizide und Fungizide sowie für Pyrimidylisocyanate (US 37 72 313) verwendet werden.

Beispiel 1

5,5-Dichlor-4,5-dihydro-6-hydroxy-2-trichlormethyl-pyrimidin-4-on

In eine Suspension von 63,1 g (0,5 mol) 4,6-Dihydroxy-2-methylpyrimidin in 500 ml o-Dichlorbenzol wurden bei 120 bis 125°C 210 g Chlor eingeleitet. Nach 5 bis 6 h des Einleitens lag eine Lösung vor. Man rührte noch 1 h bei 125°C nach, ließ abkühlen und 12 h stehen und saugte dann die ausgefallenen Kristalle bei 20°C ab und wusch mit wenig Petroleumbenzin nach.
Ausbeute: 132 g (0,442 mol), 89 % der Theorie.

Ein Teil wurde bei 150°C Badtemperatur bei 0,01 Torr sublimiert. Man erhielt farblose Kristalle vom Schmelzpunkt 150 bis 160°C.

| | | | | | |
|---|---|---|---|---|---|
| $C_5HCl_5N_2O_2$ | Ber. | C 20,13 | H 0,34 | N 9,40 | Cl 59,40 |
| (298,34) | Gef. | C 19,9 | H 0,7 | N 9,4 | Cl 59,40 |

Massenspektrum: m/e = 296 ($M^+$), Basispeak m/e = 110 ($Cl_2CCO$)

Beispiel 2

2-Trichlormethyl-4,5,6-trichlorpyrimidin

In einem 1-ltr.-Vierhalskolben mit Rührer, Thermometer, Gaseinleitungsrohr und Rückflußkühler mit Blasenzähler

wurden 63,1 g (0,5 mol) 4,6-Dihydroxy-2-methylpyrimidin in 500 ml o-Dichlorbenzol vorgelegt. Bei 130°C Sumpftemperatur wurden in 5 h 238 g Chlor eingeleitet, wonach eine Lösung vorlag.
Es wurde noch 1 h bei 130°C gerührt.
Danach wurden bei 20°C 13,8 g (0,1 mol) $PCl_3$ und 1,5 g Triethylamin zugesetzt. Dann wurden, ab 60°C beginnend, bis 115°C 123,6 g (0,9 mol) $PCl_3$ und 35,5 g (0,5 mol) Chlor simultan zudosiert. Nach dem Eindosieren wurde noch 2 h bei 120°C nachgerührt, bis die Gasentwicklung beendet war.
Nachdem Phosphoroxychlorid und o-Dichlorbenzol abdestilliert waren, wurden bei einer Kopftemperatur von 140°C und 2,5 mbar 130,1 g 2-Trichlormethyl-4,5,6-trichlorpyrimidin überdestilliert. Das entspricht einer Ausbeute von 87 % der Theorie. Schmp.: 63 bis 64°C.

Beispiel 3

In einem 1l-Vierhalskolben mit Rührer, Thermometer, Gaseinleitungsrohr, Rückflußkühler mit Blasenzähler wurden 126,1 g (1 mol) 4,6-Dihydroxy-2-methylpyrimidin in 500 ml o-Dichlorbenzol vorgelegt und auf 120°C aufgeheizt. Innerhalb von 6h wurden bei 120-130°C 420 g Chlor eingeleitet, wonach eine Lösung vorlag.
Es wurde noch 1h bei 120°C nachgerührt. Dann wurden 3 g Triethylamin und 27,6 g (0,2 mol) Phosphortrichlorid zugesetzt und bei 110-120°C wurden 247,2 g (1,8 mol) Phosphortrichlorid und 71 g Chlor gleichzeitig in ca.

45 Minuten zudosiert. Es wurde noch 2 h bei 120°C bis zum Ende der Gasentwicklung nachgerührt, danach wurde durch Destillation aufgearbeitet. Nach dem Abdestillieren von Phosphoroxychlorid und o-Dichlorbenzol wurden 264,7 g (88 %) 2-Trichlormethyl-4,5,6-trichlorpyrimidin erhalten.

Beispiel 4

2-Trichlormethyl-4,5,6-trichlorpyrimidin

In einem 500 ml-Vierhalskolben mit Rührer, Thermometer, Gaseinleitungsrohr, Tropftrichter und Rückflußkühler mit Blasenzähler wurden 149,2 g (0,5 mol) 5,5-Dichlor-4,5-dihydro-6-hydroxy-2-trichlormethyl-pyrimidin-4-on in 230 g Phosphoroxychlorid vorgelegt und auf 60°C erwärmt, wobei schwache Gasentwicklung zu beobachten war. Nach Zusetzen von 1,5 g Triethylamin wurden innerhalb von 1 h 137,4 g (1 mol) Phosphortrichlorid und 35,5 g (0,5 mol) $Cl_2$ gleichzeitig eindosiert, wobei auf bis 115°C erwärmt wurde. Es wurde noch 2 h bei 120°C nachgerührt, bis die Gasentwicklung beendet war. Nach dem Abdestillieren von Phosphoroxychlorid (ca. 370 g) wurden bei einer Kopftemperatur von 140°C und 2,5 mbar 142,8 g 2-Trichlormethyl-4,5,6-trichlorpyrimidin überdestilliert, das entspricht einer Ausbeute von 95 % d. Th.

Beispiel 5

In einem 500 ml Vierhalskolben mit Feststoffdosiermaschine, Gaseinleitungsrohr, mechanischem Rührer, Tropftrichter, Thermometer, Rückflußkühler und Blasenzähler wurden 416 g Phospohoroxychlorid vorgelegt, auf 50°C erwärmt und mit Chlor-Gas gesättigt. Bei 50°C wurden dann gleichzeitig in 170 min 63,1 g 4,6-Dihydroxy-2-methylpyrimidin mit der Feststoffdosiermaschine und 207 g Chlor eingeleitet. Danach wurde noch 1,5 h bei 80°C gerührt und es wurden 1,5 g Triethylamin und 13,7 g Phosphortrichlorid zugetropft. Nach Aufheizen auf Rückfluß-Temperatur (103°C) wurden innerhalb 1 h 123,7 g Phosphortrichlorid und 35,8 g Chlor gleichzeitig eindosiert. Nach Eintropfen des $PCl_3$ wurden innerhalb von 18 min noch 35,2 g Chlor nachdosiert. Es wurde noch 75 min bei Rückfluß bis zum Ende der Gasentwicklung gerührt und dann durch Destillation aufgearbeitet. Es wurden 539,2 g Phosphoroxychlorid und 147,7 g 98,5 %iges 4,5,6-Trichlor-2-trichlormethylpyrimidin (96,7 % der Theorie) erhalten.

Beispiel 6

In einer Apparatur wie in Beispiel 5 wurden 103 g Phosphoroxychlorid vorgelegt, auf 50°C erwärmt und mit Chlor gesättigt. Innerhalb von 143 min wurden simultan 63,1 g 4,6-Dihydroxy-2-methylpyrimidin und 183 g Chlor eingeleitet. Danach wurde die Suspension auf 65°C erwärmt und weiter Chlor eingeleitet, worauf eine gelbe Paste vorlag. Die Temperatur wurde auf

75-80°C erhöht, worauf eine gut rührbare Suspension erhalten wurde. Insgesamt wurden während des Aufheizens 54,5 g Chlor nachdosiert. Man ließ noch 20 min bei 80°C reagieren und setzte dann 1,5 g Triethylamin zu. Dann wurden in 7 min 13,7 g Phosphortrichlorid zugetropft und die Suspension wurde auf 106°C erhitzt, worauf 123,7 g Phosphortrichlorid und 35,6 g Chlor simultan eindosiert wurden. Danach wurde noch 34 min bis zur Chlor-Sättigung 23,4 g Chlor nachdosiert. Es wurde noch 17 min bei Rückfluß gerührt und dann destilliert. Es wurden 217 g Phosphoroxychlorid und 143,6 g 95,9 %iges 4,5,6-Trichlor-2-trichlormethylpyrimidin (91,6 % der Theorie) erhalten.

Beispiel 7

In einem 1l Vierhalskolben mit Rührer, Thermometer, Claisenaufsatz, Trockeneiskühler mit Blasenzähler, Einleitrohr und heizbarem Tropftrichter wurden 100 ml o-Dichlorbenzol und 2,8 g Triphenylphosphinoxid vorgelegt. Bei 150°C Sumpftemperatur wurden dann innerhalb von 7h simultan 115 g Phosgen und eine auf 100°C erwärmte Lösung von 5,5-Dichlor-4,5-dihydro-6-hydroxy-2-trichlormethyl-pyrimidin-4-on in 500 ml ortho-Dichlorbenzol zudosiert, die durch Chlorierung aus 63,1 g (0,5 mol) 4,6-Dihydroxy-2-methylpyrimidin mit 238 g Chlor bei 100°C hergestellt worden war.

Nach der Dosierung wurde noch 1h bei Rückfluß gerührt, dann das überschüssige Phosgen bei 20mbar abgezogen.

Nach Abdestillieren von o-Dichlorbenzol und Destillation über eine Vigreux-Kolonne wurden 126,3 g 92 %iges 4,5,6-Trichlor-2-trichlormethylpyrimidin (77,3 % der Theorie bezogen auf 4,6-Dihydroxy-2-methylpyrimidin) erhalten.

Patentansprüche

1. 5,5-Dichlor-4,5-dihydro-6-hydroxy-2-trichlormethyl-pyrimidin-4-on.

2. Verfahren zur Herstellung von 5,5-Dichlor-4,5-dihydro-6-hydroxy-2-trichlormethyl-pyrimidin-4-on, dadurch gekennzeichnet, daß man 4,6-Dihydroxy-2-methylpyrimidin oder chlorierte Folgestufen mit einem Chlorierungsmittel umsetzt.

3. Verfahren nach Anspruch 2, dadurch gekennzeichnet, daß man als chlorierte Folgestufe 5-Chlor-4,6-dihydroxy-2-methylpyrimidin einsetzt.

4. Verfahren nach Ansprüchen 2 und 3, dadurch gekennzeichnet, daß man mindestens stöchiometrische Mengen Chlorierungsmittel verwendet.

5. Verfahren nach den Ansprüchen 2 bis 4, dadurch gekennzeichnet, daß man die Chlorierung in Gegenwart eines Lösungs- oder Suspensionsmittels durchführt.

6. Verfahren nach den Ansprüchen 2 bis 5, dadurch gekennzeichnet, daß man die Chlorierung in Gegenwart eines Chlorierungskatalysators durchführt.

7. Verfahren zur Herstellung von 2-Trichlormethyl-4,5,6-trichlorpyrimidin, dadurch gekennzeichnet,

daß man das 5,5-Dichlor-4,5-dihydro-6-hydroxy-2-trichlormethyl-pyrimidin-4-on mit einem Säurechlorid, gegebenenfalls in Gegenwart eines Halogens der Formel

XCl

worin

X Chlor, Brom oder Iod bedeutet,

und gegebenenfalls in Gegenwart eines Katalysators umsetzt.

8. Verfahren nach Anspruch 7, dadurch gekennzeichnet, daß man als Katalysator ein Amin verwendet.

9. Verfahren zur Herstellung von 2-Trichlormethyl-4,5,6-trichlorpyrimidin dadurch gekennzeichnet, daß man 4,6-Dihydroxy-2-methyl-pyrimidin oder chlorierte Folgestufen ohne Isolierung von Zwischenstufen zunächst mit einem Chlorierungsmittel umsetzt und anschließend mit einem Säurechlorid, gegebenenfalls in Gegenwart eines Halogens der Formel X-Cl, worin X = Chlor, Brom oder Iod bedeutet, und gegebenenfalls in Gegenwart eines Katalysators.